# EUROPEAN PATENT APPLICATION

(11) **EP 4 613 856 A1**
(43) Date of publication of application: **10.09.2025**
(21) Application number: 23884084.7
(22) Date of filing: 10.04.2023
(51) Int. Cl.: C12N 9/42, C12N 15/56, A23K 20/189

(54) **XYLANASE MUTANT HAVING SPECIFIC ACTIVITY**

(30) Priority: 31.10.2022 CN 202211341756
(71) Applicant: Qingdao Vland Biotech Group Co., Ltd., Qingdao, Shandong 266100 (CN)
(72) Inventor: WU, Xiuxiu, Qingdao, Shandong 266100 (CN); LI, Xinpei, Qingdao, Shandong 266100 (CN); CHEN, Gang, Qingdao, Shandong 266100 (CN)
(74) Representative: Boult Wade Tennant LLP
(86) International application number: PCT/CN2023/087234
(87) International publication number: WO 2024/093141

(57) **Abstract**

A xylanase mutant having a high specific activity. The mutant comprises at least one mutation site of T69A/H, V85D, N88I/M/V/F/H/Y, V108L/M/T/H/I, I112V, H131L/I/W/Y/F, T149V/I/K/L/M/N/R/S/W/Y, D154M/Q/A/I/L/N/S/T/W, N160K/I/T, Q167T, S178A, A179D/M/K/L/Q/R/S/W/E, E185K, K186D and T190D/S. The specific activity of the xylanase mutant is significantly higher than the specific activity of wild-type xylanase, and the xylanase mutant can be widely used in the field of feed.

## Description

This application claims the priority of Chinese Patent Application No. 202211341756.3, filed with the China National Intellectual Property Administration on October 31, 2022, and titled with "XYLANASE MUTANT HAVING SPECIFIC ACTIVITY", the disclosure of which is hereby incorporated by reference in its entirety.

### FIELD

The present invention relates to the technical field of genetic engineering and protein engineering, in particular to a xylanase mutant with a high specific activity and use thereof.

### BACKGROUND

Xylan is a polysaccharide composed of five-carbon sugars and is an important component of plant hemicellulose, accounting for one-third of the total amount of plant carbohydrates. It is the second abundant renewable material resource in nature after cellulose. It is found mainly in the cell wall and also in almost all parts of the plants.

Xylanase is a general term for a class of enzymes that can degrade xylan into oligosaccharides or xylose. The complete enzymolysis of one xylan molecule requires several steps of enzymatic reactions, wherein two enzymes of β-1, 4-xylanase (1,4-β-D-xylanohydrolase, EC 3.1.2.8) and β-xylosidase (1,4-β-D-xylanxylohydrolase, EC 3.2.1.37) act on its backbone chain. In general, the former acts on the glycosidic bonds within the backbone chain of xylan to decompose it into oligosaccharides, and the latter acts on the ends of the oligosaccharides to release xylose.

Many microorganisms can produce xylanases. The available information about the biochemical properties of xylanases mostly from studies on bacterial and fungal xylanases. Xylanases produced by bacteria can be broadly categorized into two groups: high molecular weight xylanases with acid resistance and low molecular weight xylanases with alkali resistance. However, such a difference is not observed in fungi, but low molecular weight xylanases are also alkali-resistant.

Xylan in feeds is hard to be digested by monogastric animals. Xylan also binds a large amount of water, results in increasing the volume and viscosity of the chyme, reducing the interaction of the nutrients and the endogenous enzyme in the animal's digestive tract, and thereby impeding the digestion and absorption of nutrients, especially fats and proteins, and decreasing the digestive utilization rate of feeds. Research results have shown that the addition of xylanases to feeds can significantly reduce the size of the arabinoxylan molecule by decomposing it into xylooligosaccharides with a low degree of polymerization, thereby improving the properties of feeds, and eliminating or reducing the anti-nutritional effects caused by increased viscosity.

Xylanase has been studied for use in the production of wine and Japanese barley shochu. Japanese researchers used the acid-resistant xylanase Xy1C for brewing Japanese barley shochu and found that this enzyme can help to improve fermentation efficiency and increase alcohol yield.

Xylanase has been recognized for its application value in fields such as papermaking, foods, energy, feeds and environment. However, it is difficult to produce xylanase at a large scale due to its low expression level in natural materials and to purify the corresponding products. Moreover, xylanase has some properties that cannot fully meet the requirements of the application. With the development of genetic engineering technology, current research has focused on improving the expression level of xylanase using a bioreactor and modifying xylanase gene at the molecular level to solve some defects (e.g. enzyme specific activity, stress resistance, pH and thermal stability) by genetic engineering.

The higher the specific activity of xylanase is, the lower the production cost and price will be, which is more conducive to its wide application. The screening of xylanases with high specific activity is therefore the objective towards which the researchers in this field are working.

### SUMMARY

An objective of the present invention is to provide a xylanase mutant with high specific activity. The mutant has a significantly improved specific activity compared to its wild type counterpart, which facilitates its wide use in the industrial field.

In order to achieve the above objective of the present invention, provided are the following technical solutions.

The present invention provides a xylanase mutant comprising an amino acid sequence having at least 90% identity to SEQ ID NO: 1, wherein compared with SEQ ID NO: 1, the xylanase mutant comprises at least one amino acid substitution at a position selected from the group consisting of 69, 85, 88, 108, 112, 131, 149, 154, 160, 167, 178, 179, 185, 186 and 190 of SEQ ID NO: 1.

In some embodiments of the present invention, the xylanase mutant comprises an amino acid sequence having at least 91%, 92%, 93%, 94%, 95%, 96%, 97%, 98%, or at least 99% identity to SEQ ID NO: 1.

In some specific embodiments, the xylanase mutant comprises an amino acid sequence having at least 99.1%, 99.2%, 99.3%, 99.4%, 99.5%, 99.6%, 99.7%, 99.8%, or at least 99.9% identity to SEQ ID NO: 1.

In some embodiments of the present invention, the xylanase mutant comprises at least one amino acid substitution selected from the group consisting of T69A/H, V85D, N88I/M/V/F/H/Y, V108L/M/T/H/I, I112V, H131L/I/W/Y/F, T149V/I/K/L/M/N/R/S/W/Y, D154M/Q/A/I/L/N/S/T/W, N160K/I/T, Q167T, S178A, H179D/M/K/L/Q/R/S/W/E, E185K, K186D and T190D/S.

In some embodiments of the present invention, the xylanase mutant comprises an amino acid substitution or a combination thereof selected from the group consisting of T69A, T69H, V85D, N88I, N88M, N88V, N88F, N88H, N88Y, V108L, V108M, V108T, V108H, V108I, I112V, H131L, H131I, H131W, H131Y, H131F, T149V, T149I, T149K, T149L, T149M, T149N, T149R, T149S, T149W, T149Y, D154M, D154Q, D154A, D154I, D154L, D154N, D154S, D154T, D154W, N160K, N160I, N160T, Q167T, S178A, H179D, H179M, H179K, H179L, H179Q, H179R, H179S, H179W, H179E, E185K, K186D, T190D, T190S,
N88M/H131F, N88M/H131Y, N88M/D154L, N88I/D154L, V108I/E185K, V108I/K186D, V108I/H179L, V108I/E185M, V108I/H179T, V108I/H179Q, V108I/H179R, V108I/H179S, V108I/H131Y, H131F/H179L, H131F/H179Q, H131F/D154L, H131F/D154S, H131F/D154I, H131F/D154W, T149V/H179R, T149V/H179Q, T149I/H179L, D154M/N160I, D154M/N160T, D154W/N160T, D154W/N160I, N160K/H179Q, N160K/H179R, N160K/H179L, N160K/H179S, E185K/K186D,
N88M/H131F/T190S, N88M/H131Y/H179L, N88I/D154L/E185K, N88M/H131F/H179L, V108I/E185K/K186D, V108I/H179L/K186D, V108I/H179L/K186K, V108I/H131Y/179L, V108I/H131Y/T190S, H131F/H179L/K186D, H131F/H179L/K186K, H131F/160T/H179L, H131F/N160I/H179L, H131F/H179L/K186R, H131L/D154M/N160T, D154M/N160T/H179L, D154M/N160I/H179Q, D154M/N160I/H179L, D154M/N160I/T190S, D154M/N160I/T190E, D154M/N160I/H179M, D154M/N160I/H179E, D154M/N160I/H179W, E185K/K186D/T190L, E185K/K186D/T190D, E185K/H179L/K186D, E185K/H179Q/K186D,
N88M/H131F/D154L/T190S, N88M/H131F/D154M/T190S,
N88M/H131F/D154I/T190S, N88M/H131Y/D154M/H179L,
N88I/D154L/H179L/E185K, N88I/D154L/H179Q/E185K,
N88I/D154L/H179R/E185K, V108I/H179L/E185K/K186D,
V108I/H179Q/E185K/K186D, V108I/H179K/E185K/K186D,
V108I/H179M/E185K/K186D, V108I/H131Y/H179L/T190S,
V108I/H131Y/H179L/T190D, H131F/H179L/K186K/T190D,
H131F/N160T//H179LT190D, H131F/N160I/H179L/T190D,
H131F/H179L/K186R/T190S, D154M/N160T/H179L/T190D,
D154M/N1601/H179L/T190S, T69H/N88M/H131F/D154L/T190S,
T69H/N88M/H131F/D154M/T190S, T69A/N88M/H131F/D154I/T190S,
T69A/N88M/H131Y/D154M/H179L, T69H/N88I/D154/H179L/E185K,
N88I/V108I/D154L/H179Q/E185K, N88I/V108L/D154L/H179R/E185K,
V108I/H179Q/E185K/K186D/T190D, V108I/H179K/E185K/K186D/T190D,
V108I/H179M/E185K/K186D/T190S, H131Y/D154A/N160I/H179L/T190S,
V108I/E185K/K186D/H179K/T190D, N88M/V108L/H131Y/D154M/H179L,
T69H/N88F/H131F/D154L/H179S/T1905,
T69H/N88M/H131F/D154M/H179S/T190S,
T69A/N88V/V108I/H131F/D154I/T190S,
T69A/N88M/V108H/H131Y/D154M/H179L,
T69H/N88I/D154L/H179L/E185K/K186D,
N88I/V108I/D154L/N160K/H179Q/E185K,
N88I/V108L/D154L/H179R/E185K/T190S,
T69H/N88I/D154L/H179R/E185K/K186D,
T69H/N88I/D154L/H179Q/E185K/K186D,
T69H/N88I/D154L/H179S/E185K/K186D,
V108I//N160T/E185K/H179Q/K186D/T190D,
V108I/N160T/H179K/E185K/K186D/T190D,
V108I/N160T/H179M/185K/K186D/T190S,
V108H/H131Y/D154A/N160I/H179L/T190S,
V108I/N160T/E185K/K186D/H179K/T190D,
N88M/V108L/H131Y/T149V/D154M/H179L,
T69H/N88F/H131F/D154L/N160K/H179S/T190S,
T69H/N88M/V108I/H131F/D154M/H179S/T190S,
T69A/N88V/V108I/H131F/D154I/N1601/T190S,
T69A/N88M/V108H/H131Y/D154M/Q167T/H179L,
T69H/N88I/D154L/Q167T/H179L/E185K/K186D,
N88I/V108I/D154L/N160K/S178A/H179Q/E185K,
N88I/V108L/D154L/S178A//H179Q/E185K/T190S,
T69H/N88I/D154L/H179R/E185K/K186D/T190D,
T69H/N88I/D154L/H179Q/E185K/K186D/T190D,
T69H/N88I/D154L/S178A/H179S/E185K/K186D,
V108I//N160T/S178A/H179Q/E185K/K186D/T190D,
V108I/N160T/S178A/H179K/E185K/K186D/T190D,
V108I/D154T/S178A/H179M/E185K/K186D/T190S,
T69H/N88M/V108I/H131F/D154M/S178A/H179S/T190S,
T69A/N88V/V108I/H131F/D154I/N1601/S178A/T190S,
T69A/N88M/V108H/H131Y/D154M/Q167T/S178A/H179L,
T69H/N88I/D154L/Q167T/S178A/H179L/E185K/K186D,
N88I/V108I/D154L/N160K/S178A/H179Q/E185K/T190D,
N88I/V108L/I112V/D154L/S178A//H179R/E185K/T190S,
T69H/N88I/I112V/D154L/H179R/E185K/K186D/T190D,
T69H/N88I/I112V/D154L/H179Q/E185K/K186D/T190D,
T69H/N88I/I112V/D154L/S178A/H179S/E185K/K186D,
V108I//N160T/Q167T/S178A/H179W/E185K/K186D/T190D,
V108I/N160T/Q167T/S178A/H179K/E185K/K186D/T190D,
N88M/V108H/I112V/H131Y/D154A/N1601/Q167T/H179L/T190S,
T69H/N88M/V108I/I112V/N160T/H179K/E185K/K186D/T190D,
T69H/N88M/V108L/I112V/H131Y/T149V/D154M/H179L/T190D,
V85D/V108I/I112V/D154T/Q167T/E185K/K186D/H179M/T190S,
V85D/N88M/V108H/I112V/H131Y/D154A/N1601/Q167T/H179L/T190S,
V85D/T69H/N88M/V108I/I112V/N160T/H179K/E185K/K186D/T190D,
V85D/T69H/N88M/V108L/I112V/H131Y/T149V/D154M/H179L/T190D,
V85D/V108I/I112V/D154T/Q167T/H179M/E185K/K186D/T190S,
T69H/N8SI/I112V/D154L/Q167T/H179Q/E185K/K186D/T190D,
T69H/N88I/I112V/D154L/Q167T/S178A/H179S/E185K/K186D,
V108I/I112V/N160T/Q167T/S178A/H179Q/E185K/K186D/T190D,
T69H/V85D/N88M/V108H/I112V/H131Y/D154A/N1601/Q167T/H179L/T190S,
T69HN85D/N88M/V108I/I112V/N160T/Q167T/H179K/E185K/K186D/T190D,
T69H/V85D/N88M/V108L/I112V/H131Y/T149V/D154M/Q167T/H179L/T190D,
T69H/V85D/I112V/H131W/T149L/D154L/Q167T/H179R/E185K/K186D/T190D,
T69H/V85D/N88I/I112V/H131Y/D154L/Q167T/S178A//H179Q/E185K/K186D/T190 D,
T69H/V85D/N88I/V108L/I112V/D154L/Q167T/S178A/H179S/E185K/K186D/T190D,
T69H/V85D/N88IV108I/I112V/N160T/Q167T/S178A/H179Q/E185K/K186D/T190D, and
T69H/V85D/N88I/I112V/H131W/T149L/D154L/Q167T/H179R/E185K/K186D/T190 D.

The present invention further provides a DNA molecule encoding the xylanse mutants above.

The present invention further provides a recombinant expression vector comprising the DNA molecule.

The present invention further provides a host cell comprising the recombinant expression vector.

After transforming the vector into the host cells, the obtained recombinant xylanase mutants show significant increase in specific activity.

In some embodiments of the present invention, the host cell is *Pichia pastoris.*

In some embodiments of the present invention, the host cell is *Trichoderma reesei.*

The present invention further provides use of the xylanse mutants in the field of feeds.

The present invention provides a xylanase mutant comprising at least one point mutation selected from the group consisting of T69A/H, V85D, N88I/M/V/F/H/Y, V108L/M/T/H/I, I112V, H131L/I/W/Y/F, T149V/I/K/L/M/N/R/S/W/Y, D154M/Q/A/I/L/N/S/T/W, N160K/I/T, Q167T, S178A, H179D/M/K/L/Q/R/S/W/E, E185K, K186D and T190D/S, on the basis of the wild-type xylanase PT. The specific activity of the xylanase mutant containing a single point mutation provided by the present invention is generally increased by 10.1%-34.2%, compared with the wild-type xylanase PT. Among them, the xylanase mutant containing the single point mutation of H179L has the highest specific activity of 3033 U/mg, achieving a surprising technical effect.

In summary, the specific activity of the xylanase mutants provided by the present invention are significantly increased, thereby contributing to the reduction of the production cost of the xylanase and promoting its wide use in the industrial field.

### DETAILED DESCRIPTION

The present invention provides a xylanase mutant, a preparation method therefor and use thereof, a DNA molecule encoding the xylanase mutant, a vector and a host cell. Those skilled in the art can learn from the content of the present invention and make appropriate improvement on the process parameters to achieve the present invention. It should be particularly noted that all similar substitutions and modifications are obvious to those skilled in the art, and they are deemed to be included in the present invention. The method and the application of the present invention have been described through the preferred embodiments. It is obvious that those skilled in the art can change or appropriately modify and combine the method and application described herein without departing from the content, spirit and scope of the present invention to realize and apply the technology of the present invention.

The present invention adopts conventional techniques and methods used in the fields of genetic engineering and molecular biology, such as the methods described in MOLECULAR CLONING: A LABORATORY MANUAL, 3nd Ed. (Sambrook, 2001) and CURRENT PROTOCOLS IN MOLECULAR BIOLOGY (Ausubel, 2003). These general references provide definitions and methods known to those skilled in the art. However, those skilled in the art can adopt other conventional methods, experimental schemes, and reagents in the field on the basis of the technical solutions described in the present invention, and are not limited to the particular embodiments of the present invention. For example, the present invention may use the following experimental materials and reagents.

Strains and Vectors: *Escherichia coli* DH5α, *Pichia pastoris* GS115, pPIC9k vector, Amp (ampicillin) and G418 were purchased from Invitrogen.

Enzymes and Kits: PCR enzymes and ligases were purchased from Takara, restriction enzymes were purchased from Fermentas, plasmid extraction kits and gel purification recovery kits were purchased from Omega, and GeneMorph II random mutagenesis kits were purchased from Beijing Biomars Biological Technology Co., Ltd.

The formula of medium is as follows:
*Escherichia coli* culture medium (LB medium): 0.5% yeast extract, 1% peptone, 1% NaCl, pH 7.0.

Yeast culture medium (YPD medium): 1% yeast extract, 2% peptone, 2% glucose.

Yeast screening medium (MD plate): 2% peptone, 2% agarose.

BMGY medium: 2% peptone, 1% yeast extract, 100 mM potassium phosphate buffer (pH 6.0), 1.34% YNB, 4×10⁻⁵ % biotin, 1% glycerol.

BMMY medium: 2% peptone, 1% yeast extract, 100 mM potassium phosphate buffer (pH 6.0), 1.34% YNB, 4×10⁻⁵ % biotin, 0.5% methanol.

LB-AMP medium: 0.5% yeast extract, 1% peptone, 1% NaCl, 100 µg/mL ampicillin, pH 7.0.

LB-AMP plate: 0.5% yeast extract, 1% peptone, 1% NaCl, 1.5% agar, 100 µg/mL ampicillin.

The raw materials and reagents used in the present invention for the xylanase mutant, the preparation method therefor and use thereof, the DNA molecule encoding the xylanase mutant, the vector and the host cell are all commercially available.

The present invention is further illustrated below in conjunction with examples.

### Example 1 Construction of recombinant plasmid

The synthesized xylanase gene PT from eukaryote (genus *neocallimastix, phylum chytridiomycota*) (GeneBank accession number: MK138894.1) was optimized according to the codon preference of *Pichia pastoris.* A 6-base sequence of GAATTC (EcoRI restriction site) was added upstream of the start codon ATG, and a sequence of GCGGCCGC (NotI restriction site) was added downstream of the stop codon TAA. The optimized nucleotide sequence was synthesized by Shanghai Generay Biotech Co., Ltd to obtain the xylanase gene named as PT, its amino acid sequence is set forth in SEQ ID NO: 1 and the coding nucleotide sequence is set forth in SEQ ID NO: 2.

The xylanase gene was digested with the restriction endonucleases EcoRI and NotI (Fermentas), and the pPIC9K plasmid was also digested with the restriction endonucleases EcoRI and NotI. The products after digestion were purified using a gel purification kit, and the two products were ligated using T4 DNA ligase (Fermentas). The ligation products were transformed into *E*. *coli* DH5α (Invitrogen). The clones were selected by ampicillin. Several clones were sequenced (Sangon) to ensure accuracy.

Plasmids were purified from the *E. coli* clones with correct sequencing results by using a kit for the small scale preparation of plasmid (Omega) to obtain one recombinant plasmid, which was named pPIC9K-PT.

### Example 2 Screening of mutants with high specific activity

In order to further improve the specific activity of xylanase PT, the structure of this protein was analyzed. This protein is a xylanase of the glycoside hydrolase family 11 (GH11), which displays a β-jelly roll structure composed of twisted sheets and resembles a partly-closed right hand shape. Two catalytic residues are located in cavities that are formed by highly twisted β-pleated sheets and are capable of accommodating xylan chains. Without destroying the secondary structure and active center of the protein, a large number of mutations of the enzyme were screened by directed evolution technique in the present invention.

### 1.1 Design of PCR primers PT-F1 and PT-R1:

PT-F1: GGCGAATTCCAAAGTTTCTGTAGTTCAGCTTCTC (as set forth in SEQ ID NO: 3, the underlined letters represent the recognition site of the restriction endonuclease EcoRI);
PT-R1: ATAGGCGGCCGCTTATCATTAATCACCAATGTAAACCTT (as set forth in SEQ ID NO: 4, the underlined letters represent the recognition site of the restriction endonuclease NotI).

The PT gene (SEQ ID NO: 2) was used as a template, and the primers above were used to perform PCR amplification by GeneMorph II Random Mutagenesis Kit (Biomars), and the PCR product was recovered from gel. After being digested with EcoRI and NotI, the PCR product was ligated with pET21a vector digested with the same enzymes. The ligation product was transformed into *E*. *coli* BL21 (DE3) and then the bacteria were spread on LB+Amp plate and culturing upside down at 37°C. After colonies of transformants appeared, single colonies were picked one by one with toothpicks and transferred to a 96-well plate. Each well of the plate was added with 150 µl of LB+Amp medium containing 0.1 mM IPTG, followed by culturing at 37°C, 220 rpm for about 6 h. The culture was centrifuged and the supernatant was discarded. The bacteria cells were resuspended in buffer, and frozen and thawed repeatedly to break the cell wall and obtain *E. coli* cell lysate containing xylanase.

30 µl of lysate was taken from each well and transferred to two new 96-well plates respectively. 30 µl of substrate was added to each well of one plate, and the reaction was carried out at 37°C for 30 min. The reducing sugar was determined by DNS method. 150 µl of Coomassie brilliant blue solution was added to each well of the other plate, and this plate was left to stand for 10 min. The protein concentration was determined by Coomassie brilliant blue-binding method (Bradford assay), and the enzyme activity and protein concentration in different mutants were calculated respectively. Finally, the mutations that significantly increase the specific activity of PT without affecting its original enzymatic properties were screened out from more than 20,000 transformants and listed as follows: T69A, T69H, V85D, N88I, N88M, N88V, N88F, N88H, N88Y, V108L, V108M, V108T, V108H, V108I, I112V, H131L, H131I, H131W, H131Y, H131F, T149V, T149I, T149K, T149L, T149M, T149N, T149R, T149S, T149W, T149Y, D154M, D154Q, D154A, D154I, D154L, D154N, D154S, D154T, D154W, N160K, N160I, N160T, Q167T, S178A, H179D, H179M, H179K, H179L, H179Q, H179R, H179S, H179W, H179E, E185K, K186D, T190D and T190S.

On the basis of wild-type xylanase PT, the present invention provides mutants with single point mutation of each mutation site above respectively.

The present invention further provides a mutant comprising a combination of the mutation sites, and the combination comprises at least 2, at least 3, at least 4, at least 5, at least 6, at least 7, at least 8, at least 9, at least 10, at least 11 or at least 12 mutation sites listed above.

### Example 3 Expression of xylanase in Pichia pastoris

### 3.1 Construction of expression vector

According to the codon preference of *Pichia pastoris,* the gene sequences of xylanase PT and mutants thereof were optimized respectively. The genes were synthesized by Shanghai Generay Biotechnology Co., Ltd., and two restriction enzyme cutting sites of EcoRI and NotI were added to the 5' and 3' ends of the synthetic sequence, respectively.

Following the method described in Example 1, the synthesized gene sequences of the xylanase and the mutants thereof were separately digested with EcoRI and NotI, and then ligated with the pPIC-9K vector (digested with the same enzymes) at 16°C overnight. The ligation product was transformed into *E. coli* DH5a and then spread on LB+Amp plate and culture upside down at 37°C. After transformants appeared, the positive clones were verified by colony PCR (reaction system: single clone as template; 0.5 µl of rTaq DNA polymerase, 2.0 µL of 10×Buffer, 2.0 µL of dNTPs (2.5 mM), 0.5 µl of 5'AOX primer (10 mM), 0.5 µl of 3'AOX primer, 14.5 µL of ddH₂O; reaction program: pre-denaturation at 95°C for 5min; 30 cycles of 94°C for 30 sec, 55°C for 30 sec and 72°C for 2 min; and 72°C for 10min), and correct recombinant expression plasmids were obtained after sequencing verification.

### 3.2 Construction of engineered strain of Pichia pastoris

### 3.2.1 Preparation of yeast competent cells

*Pichia pastoris* GS115 strain was activated on an YPD plate. After culturing at 30°C for 48 h, single clone of the activated GS115 was inoculated into 6 mL of YPD liquid medium and cultured at 220 rpm, 30°C for about 12 h. After that, the yeast culture was transferred into an Erlenmeyer flask containing 30 mL of YPD liquid medium and cultured at 220 rpm, 30°C for about 5 h. The culture density was measured by using an ultraviolet spectrophotometer. After the OD600 value reached to the range of 1.1-1.3, 4 mL of culture was collected into a sterilized EP tube and centrifuged at 4°C, 9000 rpm for 2 min. The supernatant was gently discarded, and the remaining supernatant was removed by absorbing with a sterilized filter paper. The cells were then resuspended in 1 mL of pre-cooled sterilized water, followed by centrifugation at 4°C, 9000 rpm for 2 min. The supernatant was gently discarded, and the cells were washed again with 1 mL of sterilized water, followed by centrifugation at 4°C, 9000 rpm for 2 min. The supernatant was gently discarded, and the cells were resuspended in 1 mL of pre-cooled sorbitol (1 mol/L), followed by centrifugation at 4°C, 9000 rpm for 2 min. The supernatant was gently discarded, and the cells were gently resuspended in 100-150 µl of the pre-cooled sorbitol (1 mol/L).

### 3.2.2 Transformation and screening

The recombinant expression plasmids constructed in Example 3.1 were respectively linearized with SacI. The linearized fragments were purified and recovered, and then they were separately transformed into *Pichia pastoris* GS115 by electroporation. The recombinant strains of *Pichia pastoris* were screened on MD plates, and then transformants with multiple copies of plasmid were screened on YPD plates containing different concentrations of geneticin (0.5 mg/mL-8 mg/mL).

The obtained transformants were separately transferred and inoculated to BMGY medium and cultured with shaking at 250 rpm, 30°C and for 1 day, then transferred to BMMY medium and cultured with shaking at 250 rpm, 30°C. 0.5% methanol was added every day to induce expression for 4 days. The cells were removed by centrifugation at 9000 rpm for 10 min to obtain supernatants after fermentation, which contained xylanase PT or xylanase mutant.

### 1. Enzyme activity assay of xylanase

### (1) Definition of enzyme activity unit of xylanase

Under a condition of 37°C and pH 5.5, the amount of enzyme required to release 1 µmol of reducing sugar per minute from a xylan solution with a concentration of 5 mg/ml is defined as one enzyme-activity unit (U).

### (2) Enzyme activity assay

2 ml of 1% xylan substrate (prepared in acetic acid-sodium acetate buffer, pH 5.5) was added to a colorimetric tube, followed by 10 min of equilibration at 37°C. 2 mL of xylanase solution (diluted with acetic acid-sodium acetate buffer (pH 5.5) and equilibrated at 37°C) was added to the substrate. This mixture was mixed well at 37°C and the precise temperature was maintained to allow a 30 min of reaction. After the reaction was over, 5 ml DNS reagent was added and mixed well to terminate the reaction. The resulting mixture was heated in boiling water bath for 5 min, cooled to room temperature with tap water, added with distilled water to adjust the volume to 25 mL, and mixed well. A standard blank sample was used as a blank control. The absorbance represented by AE was measured at 540 nm.

The calculation formula of enzyme activity is as follow: ${X}_{D} = \frac{\left[\left({A}_{E}-{A}_{B}\right)\times K+{C}_{0}\right]}{M \times t} \times N \times 1000 .$

In the formula, X_{D} represents the enzyme activity of xylanase in the enzyme solution after dilution, U/ml; *A*_{E} represents the absorbance of the enzyme reaction solution; *A*_{B} represents the absorbance of blank enzyme solution; K represents the slope of the standard curve; C₀ represents the intercept of the standard curve; M represents the molar mass of xylose, 180.2 g/mol; t represents the reaction time of enzymolysis, min; N represents the dilution multiple of enzyme solution; and 1000 is the conversion factor, 1 mmol=1000 µmol.

### (3) Results of enzyme activity assay

The enzyme activity was measured according to the above method. The results showed that the enzyme activity in the fermentation supernatant from the recombinant *Pichia pastoris* strain expressing recombinant xylanase PT or mutant was in a range of 295-1030 U/mL.

### 2. Method of measuring protein concentration

Coomassie Brilliant Blue binding method (Bradford assay) is a composite method combining colorimetric and pigment method to determine protein concentration. Coomassie Brilliant Blue G-250 is brownish-red in acidic solution, and turns blue when binds to protein. The color change conforms to Beer's law within a certain concentration range of protein, and can be measured by colorimetry at 595 nm. It shows obvious absorption within 3 to 5 minutes and is stable for at least 1 h. The absorbance is directly proportional to the protein concentration within the range of 10-1000 µg/mL.

The enzyme solution and Coomassie Brilliant Blue solution were mixed at a volume ratio of 1:5, and left to stand for 10 min. The protein concentration was determined by the Coomassie Brilliant Blue binding method (Bradford assay).

The xylanase protein concentrations in the fermentation supernatant of the above-mentioned engineered *Pichia pastoris* strains expressing wild-type xylanase or mutants thereof were measured according to the above method. The results showed that the protein concentration in the fermentation supernatant of the recombinant strain of *Pichia pastoris* expressing recombinant xylanase PT or mutant thereof constructed above was in a range of 0.125-0.335 mg/mL.

### 3. Calculation of specific activity

"Specific Activity" refers to the number of enzyme activity units in a unit weight of protein, generally expressed in U/mg protein. Generally speaking, the higher the specific activity of the enzyme, the purer the enzyme.

Calculation formula of specific activity: specific activity (U/mg) = enzyme activity (U/mL) / protein concentration (mg/mL).

The specific activity of xylanases in the fermentation supernatants of the engineered strains of *Pichia pastoris* expressing wild-type xylanase PT or mutant thereof was calculated respectively.

The specific calculation results are shown in Table 1.

**Table 1 Comparison of enzyme activity between xylanase PT and mutant thereof**

| Mutant | Specific activity (U/mg) | Mutant | Specific activity (U/mg) |
|---|---|---|---|
| Wild-type PT | 2260 | T149W | 2501 |
| T69A | 2577 | T149Y | 2497 |
| T69H | 2631 | D154M | 2566 |
| V85D | 2598 | D154Q | 2572 |
| N88I | 2783 | D154A | 2561 |
| N88M | 2816 | D154I | 2570 |
| N88V | 2557 | D154L | 2568 |
| N88F | 2613 | D154N | 2543 |
| N88H | 2511 | D154S | 2529 |
| N88Y | 2512 | D154T | 2572 |
| V108L | 2509 | D154W | 2514 |
| V108M | 2519 | N160K | 2621 |
| V108T | 2526 | N160I | 2577 |
| V108H | 2563 | N160T | 2575 |
| V108I | 2633 | Q167T | 2499 |
| I112V | 2631 | S178A | 2517 |
| H131L | 2577 | H179D | 2488 |
| H131I | 2639 | H179M | 2501 |
| H131W | 2699 | H179K | 2633 |
| H131Y | 2897 | H179L | 3033 |
| H131F | 3012 | H179Q | 2977 |
| T149V | 2629 | H179R | 2836 |
| T149I | 2561 | H179S | 2777 |
| T149K | 2517 | H179W | 2555 |
| T149L | 2539 | H179E | 2541 |
| T149M | 2563 | E185K | 2529 |
| T149N | 2537 | K186D | 2497 |
| T149R | 2515 | T190D | 2503 |
| T149S | 2522 | T190S | 2566 |

As seen from data shown in Table 1, the specific activity of the xylanase mutants containing a single point mutation provided by the present invention was generally increased by 10.1%-34.2%, compared to the wild-type xylanase PT. Among them, the xylanase mutant containing the single point mutation of H179L had the highest specific activity of 3033 U/mg, achieving an unexpected effect.

In summary, the xylanase mutants provided by the present invention have a higher specific activity and a lower cost advantage, and are more suitable for use as a feed additive compared to wild type xylanase.

The above describes in detail the xylanase mutant, the preparation method therefor and use thereof, the DNA molecule encoding the xylanase mutant, the vector and the host cell provided by the present invention. The principle and implementation of the present invention are illustrated by using specific embodiments herein. The above descriptions of the embodiments are only used to facilitate understanding of the method and the core idea of the present invention. It should be noted that, those skilled in the art can make several improvements and modifications to the present invention without departing from the principle of the present invention, and the improvements and modifications shall fall within the protection scope of the claims.

Sequence Listing

<?xml version="1.0" encoding="UTF-8"?>
<!DOCTYPE ST26SequenceListing SYSTEM "ST26SequenceListing_V1_3.dtd" PUBLIC
"-//WIPO//DTD Sequence Listing 1.3//EN">
<ST26SequenceListing productionDate="2023-04-06" softwareVersion="2.1.1"
softwareName="WIPO Sequence" fileName="OP230183.xml"
dtdVersion="V1_3"><ApplicantFileReference>OP230183</ApplicantFileReference><Ea
rliestPriorityApplicationIdentification><IPOfficeCode>CN</IPOfficeCode><Applic
ationNumberText>202211341756.3</ApplicationNumberText><FilingDate>2022-10-31</
FilingDate></EarliestPriorityApplicationIdentification><ApplicantName
languageCode="zh"> QINGDAO VLAND BIOTECH GROUP CO.,
LTD</ApplicantName><ApplicantNameLatin>QINGDAO VLAND BIOTECH GROUP
CO.,LTD</ApplicantNameLatin><InventionTitle languageCode="zh"> XYLANASE MUTANT
HAVING SPECIFIC
ACTIVITY</InventionTitle><SequenceTotalQuantity>4</SequenceTotalQuantity><Sequ
enceData
sequenceIDNumber="1"><INSDSeq><INSDSeq_length>225</INSDSeq_length><INSDSeq_mol
type>AA</INSDSeq_moltype><INSDSeq_division>PAT</INSDSeq_division><INSDSeq_feat
ure-table><INSDFeature><INSDFeature_key>source</INSDFeature_key><INSDFeature_l
ocation>1..225</INSDFeature_location><INSDFeature_quals><INSDQualifier><INSDQu
alifier_name>mol_type</INSDQualifier_name><INSDQualifier_value>protein</INSDQu
alifier_value></INSDQualifier><INSDQualifier
id="q2"><INSDQualifier_name>organism</INSDQualifier_name><INSDQualifier_value>
synthetic
construct</INSDQualifier_value></INSDQualifier></INSDFeature_quals></INSDFeatu
re></INSDSeq_feature-table><INSDSeq_sequence>QSFCSSASHSGQSVKVTGNKVGTIGGVGYELWA
DSGNNSATFYSDGSFSCTFQNAGDYLCRSGLSFDSTKTPSQIGRMKADFKLVKQNSSNVGYSYVGVYGWTRSPLVEYY
IVDNWLSPFPPGDWVGNKKHGSFTIDGAQYTVYENTRTGPSIDGDTTFNQYFSIRQQARDCGTIDISAHFDQWEKLGM
TMGKLHEAKVLGEAGNVNGGASGTADFPYAKVYIGD</INSDSeq_sequence></INSDSeq></SequenceDat
a><SequenceData
sequenceIDNumber="2"><INSDSeq><INSDSeq_length>684</INSDSeq_length><INSDSeq_mol
type>DNA</INSDSeq_moltype><INSDSeq_division>PAT</INSDSeq_division><INSDSeq_fea
ture-table><INSDFeature><INSDFeature_key>source</INSDFeature_key><INSDFeature_
location>1..684</INSDFeature_location><INSDFeature_quals><INSDQualifier><INSDQ
ualifier_name>mol_type</INSDQualifier_name><INSDQualifier_value>other
DNA</INSDQualifier_value></INSDQualifier><INSDQualifier
id="q3"><INSDQualifier_name>organism</INSDQualifier_name><INSDQualifier_value>
synthetic
construct</INSDQualifier_value></INSDQualifier></INSDFeature_quals></INSDFeatu

re></INSDSeq_feature-table><INSDSeq_sequence>caaagtttctgtagttcagcttctcactctgga
caaagtgtaaaggtaaccggcaacaaggttggaactattggtggtgttggttacgaattatgggctgatagtggtaat
aacagtgctactttctattctgatggttccttctcatgtactttccaaaatgctggggattacttatgtcgtagtggt
ctttctttcgatagtactaagaccccatctcaaattggtcgtatgaaggctgatttcaaacttgtcaaacaaaatagt
tccaatgttggttattcctatgttggtgtttacggttggactagaagtccacttgtcgaatactacattgtcgataat
tggcttagtccattcccaccaggtgattgggttggtaacaagaagcatggttctttcactattgatggtgctcaatac
actgtttatgaaaacactagaactggtccatctattgatggtgataccaccttcaatcaatacttcagtattcgtcaa
caagctcgtgattgtggtaccattgatatttctgctcactttgatcaatgggaaaagcttggtatgactatgggtaaa
ttacatgaagccaaggttttaggtgaagccggtaacgttaacggtggtgccagtggtaccgctgatttcccgtacgca
aaggtttacattggtgattaatgataa</INSDSeq_sequence></INSDSeq></SequenceData><Sequen
ceData
sequenceIDNumber="3"><INSDSeq><INSDSeq_length>34</INSDSeq_length><INSDSeq_molt
ype>DNA</INSDSeq_moltype><INSDSeq_division>PAT</INSDSeq_division><INSDSeq_feat
ure-table><INSDFeature><INSDFeature_key>source</INSDFeature_key><INSDFeature_l
ocation>1..34</INSDFeature_location><INSDFeature_quals><INSDQualifier><INSDQua
lifier_name>mol_type</INSDQualifier_name><INSDQualifier_value>other
DNA</INSDQualifier_value></INSDQualifier><INSDQualifier
id="q4"><INSDQualifier_name>organism</INSDQualifier_name><INSDQualifier_value>
synthetic
construct</INSDQualifier_value></INSDQualifier></INSDFeature_quals></INSDFeatu
re></INSDSeq_feature-table><INSDSeq_sequence>ggcgaattccaaagtttctgtagttcagcttct
c</INSDSeq_sequence></INSDSeq></SequenceData><SequenceData
sequenceIDNumber="4"><INSDSeq><INSDSeq_length>39</INSDSeq_length><INSDSeq_molt
ype>DNA</INSDSeq_moltype><INSDSeq_division>PAT</INSDSeq_division><INSDSeq_feat
ure-table><INSDFeature><INSDFeature_key>source</INSDFeature_key><INSDFeature_l
ocation>1..39</INSDFeature_location><INSDFeature_quals><INSDQualifier><INSDQua
lifier_name>mol_type</INSDQualifier_name><INSDQualifier_value>other
DNA</INSDQualifier_value></INSDQualifier><INSDQualifier
id="q5"><INSDQualifier_name>organism</INSDQualifier_name><INSDQualifier_value>
synthetic
construct</INSDQualifier_value></INSDQualifier></INSDFeature_quals></INSDFeatu
re></INSDSeq_feature-table><INSDSeq_sequence>ataggcggccgcttatcattaatcaccaatgta
aacctt</INSDSeq_sequence></INSDSeq></SequenceData></ST26SequenceListing>

## Claims

1. A xylanase mutant comprising an amino acid sequence having at least 90% identity to SEQ ID NO: 1, wherein compared with SEQ ID NO: 1, the xylanase mutant comprises at least one amino acid substitution at a position selected from the group consisting of 69, 85, 88, 108, 112, 131, 149, 154, 160, 167, 178, 179, 185, 186 and 190 of SEQ ID NO: 1,

2. The xylanase mutant according to claim 1, wherein the xylanase mutant comprises an amino acid sequence having at least 91%, 92%, 93%, 94%, 95%, 96%, 97%, 98%, or at least 99% identity to SEQ ID NO: 1.

3. The xylanase mutant according to claim 2, wherein the xylanase mutant comprises an amino acid sequence having at least 99.1%, 99.2%, 99.3%, 99.4%, 99.5%, 99.6%, 99.7%, 99.8%, or at least 99.9% identity to SEQ ID NO: 1.

4. The xylanase mutant according to claim 1, wherein the xylanase mutant comprises at least one amino acid substitution selected from the group consisting of T69A/H, V85D, N88I/M/V/F/H/Y, V108L/M/T/H/I, I112V, H131L/I/W/Y/F, T149V/I/K/L/M/N/R/S/W/Y, D154M/Q/A/I/L/N/S/T/W, N160K/I/T, Q167T, S178A, H179D/M/K/L/Q/R/S/W/E, E185K, K186D and T190D/S.

5. The xylanase mutant according to claim 4, wherein the xylanase mutant comprises at least one amino acid substitution or a combination thereof selected from the group consisting of T69A, T69H, V85D, N88I, N88M, N88V, N88F, N88H, N88Y, V108L, V108M, V108T, V108H, V108I, I112V, H131L, H131I, H131W, H131Y, H131F, T149V, T149I, T149K, T149L, T149M, T149N, T149R, T149S, T149W, T149Y, D154M, D154Q, D154A, D154I, D154L, D154N, D154S, D154T, D154W, N160K, N160I, N160T, Q167T, S178A, H179D, H179M, H179K, H179L, H179Q, H179R, H179S, H179W, H179E, E185K, K186D, T190D, T190S,
N88M/H131F, N88M/H131Y, N88M/D154L, N88I/D154L, V108I/E185K, V108I/K186D, V108I/H179L, V108I/E185M, V108I/H179T, V108I/H179Q, V108I/H179R, V108I/H179S, V108I/H131Y, H131F/H179L, H131F/H179Q, H131F/D154L, H131F/D154S, H131F/D154I, H131F/D154W, T149V/H179R, T149V/H179Q, T149I/H179L, D154M/N160I, D154M/N160T, D154W/N160T, D154W/N160I, N160K/H179Q, N160K/H179R, N160K/H179L, N160K/H179S, E185K/K186D,
N88M/H131F/T190S, N88M/H131Y/H179L, N88I/D154L/E185K, N88M/H131F/H179L, V108I/E185K/K186D, V108I/H179L/K186D, V108I/H179L/K186K, V108I/H131Y/179L, V108I/H131Y/T190S, H131F/H179L/K186D, H131F/H179L/K186K, H131F/160T/H179L, H131F/N160I/H179L, H131F/H179L/K186R, H131L/D154M/N160T, D154M/N160T/H179L, D154M/N160I/H179Q, D154M/N160I/H179L, D154M/N160I/T190S, D154M/N160I/T190E, D154M/N160I/H179M, D154M/N160I/H179E, D154M/N160I/H179W, E185K/K186D/T190L, E185K/K186D/T190D, E185K/H179L/K186D, E185K/H179Q/K186D,
N88M/H131F/D154L/T190S, N88M/H131F/D154M/T190S,
N88M/H131F/D154I/T190S, N88M/H131Y/D154M/H179L,
N88I/D154L/H179L/E185K, N88I/D154L/H179Q/E185K,
N88I/D154L/H179R/E185K, V108I/H179L/E185K/K186D,
V108I/H179Q/E185K/K186D, V108I/H179K/E185K/K186D,
V108I/H179M/E185K/K186D, V108I/H131Y/H179L/T190S,
V108I/H131Y/H179L/T190D, H131F/H179L/K186K/T190D,
H131F/N160T//H179LT190D, H131F/N160I/H179L/T190D,
H131F/H179L/K186R/T190S, D154M/N160T/H179L/T190D,
D154M/N160I/H179L/T190S, T69H/N88M/H131F/D154L/T190S,
T69H/N88M/H131F/D154M/T190S, T69A/N88M/H131F/D154I/T190S,
T69A/N88M/H131Y/D154M/H179L, T69H/N88I/D154/H179L/E185K,
N88I/V108I/D154L/H179Q/E185K, N88I/V108L/D154L/H179R/E185K,
V108I/H179Q/E185K/K186D/T190D, V108I/H179K/E185K/K186D/T190D,
V108I/H179M/E185K/K186D/T190S, H131Y/D154A/N160I/H179L/T190S,
V108I/E185K/K186D/H179K/T190D, N88M/V108L/H131Y/D154M/H179L,
T69H/N88F/H131F/D154L/H179S/T190S,
T69H/N88M/H131F/D154M/H179S/T190S,
T69A/N88V/V108I/H131F/D154I/T190S,
T69A/N88M/V108H/H131Y/D154M/H179L,
T69H/N881/D154L/H179L/E185K/K186D,
N88I/V108I/D154L/N160K/H179Q/E185K,
N88I/V108L/D154L/H179R/E185K/T190S,
T69H/N881/D154L/H179R/E185K/K186D,
T69H/N881/D154L/H179Q/E185K/K186D,
T69H/N881/D154L/H179S/E185K/K186D,
V108I//N160T/E185K/H179Q/K186D/T190D,
V108I/N160T/H179K/E185K/K186D/T190D,
V108I/N160T/H179M/E185K/K186D/T190S,
V108H/H131Y/D154A/N160I/H179L/T190S,
V108I/N160T/E185K/K186D/H179K/T190D,
N88M/V108L/H131Y/T149V/D154M/H179L,
T69H/N88F/H131F/D154L/N160K/H179S/T1905,
T69H/N88M/V108I/H131F/D154M/H179S/T190S,
T69A/N88V/V108I/H131F/D154I/N160I/T190S,
T69A/N88M/V108H/H131Y/D154M/Q167T/H179L,
T69H/N88I/D154L/Q167T/H179L/E185K/K186D,
N88I/V108I/D154L/N160K/S178A/H179Q/E185K,
N88I/V108L/D154L/S178A//H179Q/E185K/T190S,
T69H/N88I/D154L/H179R/E185K/K186D/T190D,
T69H/N881/D154L/H179Q/E185K/K186D/T190D,
T69H/N88I/D154L/S178A/H179S/E185K/K186D,
V108I//N160T/S178A/H179Q/E185K/K186D/T190D,
V108I/N160T/S178A/H179K/E185K/K186D/T190D,
V108I/D154T/S178A/H179M/E185K/K186D/T190S,
T69H/N88M/V108I/H131F/D154M/S178A/H179S/T190S,
T69A/N88V/V108I/H131F/D154I/N1601/S178A/T190S,
T69A/N88M/V108H/H131Y/D154M/Q167T/S178A/H179L,
T69H/N88I/D154L/Q167T/S178A/H179L/E185K/KI86D,
N88I/V108I/D154L/N160K/S178A/H179Q/E185K/T190D,
N88I/V108L/I112V/D154L/S178A//H179R/E185K/T1905,
T69H/N88I/I112V/D154L/H179R/E185K/K186D/T190D,
T69H/N88I/I112V/D154L/H179Q/E185K/K186D/T190D,
T69H/N88I/I112V/D154L/S178A/H179S/E185K/K186D,
V108I//N160T/Q167T/S178A/H179Q/E85K/K186D/T190D,
V108I/N160T/Q167T/S178A/H179K/E185K/K186D/T190D,
N88M/V108H/I112V/H131Y/D154A/N160I/Q167T/H179L/T1905,
T69H/N88M/V108I/I112V/N160T/H179K/E185K/K186D/T190D,
T69H/N88M/V108L/I112V/H131Y/T149V/D154M/H179L/T190D,
V85D/V108I/I112V/D154T/Q167T/E185K/K186D/H179M/T1908S,
V85D/N88M/V108H/I112V/H131Y/D154A/N160I/Q167T/H179L/T190S,
V85D/T69H/N88M/V108I/I112V/N160T/H179K/E185K/K186D/T190D,
V85D/T69H/N88M/V108L/I112V/H131Y/T149V/D154M/H179L/T190D,
V85D/V108I/I112V/D154T/Q167T/H179M/E185K/K186D/T190S,
T69H/N88I/I112V/D154L/Q167T/H179Q/E185K/K186D/T190D,
T69H/N88I/I112V/D154L/Q167T/S178A/H179S/E185K/K186D,
V1081/I112V/N160T/Q167T/S178A/H179Q/E185K/K186D/T190D,
T69H/V85D/N88M/V108H/I112V/H131Y/D154A/N160I/Q167T/H179L/T190S,
T69H/V85D/N88M/V108I/I112V/N160T/Q167T/H179K/E185K/K186D/T190D,
T69H/V85D/N88M/V108L/I112V/H131Y/T149V/D154M/Q167T/H179L/T190D,
T69H/V85D/I112V/H131W/T149L/D154L/Q167T/H179R/E185K/K186D/T190D,
T69H/V85D/N88I/I112V/H131Y/D154L/Q167T/S178 A/H179Q/E185K/K186D/T190D,
T69H/V85D/N88I/V108L/M112V/D154L/Q167T/S178A/H179S/E185K/K186D/T190D,
T69H/V85D/N88IV108I/I112V/N160T/Q167T/S178 A/H179Q/E185K/K186D/T190D, and
T69H/V85D/N88I/I112V/H131W/T149L/D154L/Q167T/H179R/E185K/K186D/T190D.

6. A DNA molecule encoding the xylanse mutant according to any one of claims 1 to 5.

7. A recombinant expression plasmid comprising the DNA molecule according to claim 6.

8. A host cell comprising the recombinant expression plasmid according to claim 7, wherein the host cell is a non-plant cell.

9. The host cell according to claim 8, wherein the host cell is selected from the group consisting of *Pichia pastoris* and *Trichoderma reesei.*

10. Use of the xylanse mutant according to any one of claims 1 to 5 in the field of feeds.
